# EUROPEAN PATENT APPLICATION

(11) **EP 3 944 860 A1**
(43) Date of publication of application: **02.02.2022**
(21) Application number: 20382693.8
(22) Date of filing: 30.07.2020
(51) Int. Cl.: A61K 31/58, A61K 9/48, A61P 35/00, A61P 35/04

(54) **ABIRATERONE FOR USE IN A METHOD OF TREATING CANCER**

(71) Applicant: Galenicum Health S.L.U., 08950 Esplugues de Llobregat (ES)
(72) Inventor: TORREJÓN NIETO, Javier, 08005 Barcelona (BARCELONA) (ES); GÓMEZ COELLO, Luis, 08005 Barcelona (BARCELONA) (ES); BERNAL PÉREZ, Rocío, 08005 Barcelona (BARCELONA) (ES)
(74) Representative: Pons

(57) **Abstract**

The present invention relates to a total daily amount of abiraterone, abiraterone acetate or pharmaceutically acceptable salts thereof for use in a method for treating cancer. Formulations and manufacturing processes are also described.

## Description

### FIELD

The present invention relates to a total daily amount of abiraterone, abiraterone acetate or pharmaceutically acceptable salts thereof for use in a method for treating cancer. Formulations and manufacturing processes are also described.

### BACKGROUND

Abiraterone acetate is a selective inhibitor of 17 a-hydroxylase/C17, 20-lyase (CYP17) which is known to be essential for the biosynthesis of androgens and oestrogens. CYP17 is expressed in testicular, adrenal, and prostatic tumor tissues. Said enzyme complex catalyses the conversion of pregnenolone and progesterone to their 17a-hydroxy derivatives by its 17a-hydroxylase activity, and the subsequent formation of the androgens dehydroepiandrosterone (DHEA) and androstenedione, by its C17,20 lyase activity. The androgens DHEA and androstenedione are precursors of testosterone. As a consequence, inhibition of CYP17 activity by abiraterone decreases circulating levels of testosterone and other androgens in cancer patients.

Different routes of synthesis for abiraterone acetate are known in the art, e.g. WO 93/20097, WO 95/09178 and WO 06/021777. Abiraterone acetate has the following structure:

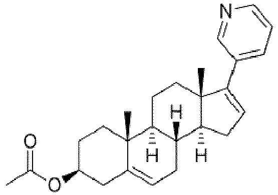

Abiraterone acetate, commercialized under the name of Zytiga^{®}, was developed by Janssen, and approved in 2011 for its use in combination with prednisone for the treatment of patients with metastatic castration-resistant prostate cancer.

Zytiga^{®} is known to exhibit a very strong food effect when administered orally. In particular, administration with food increases absorption of the drug and thus, potentially results in increased and highly variable exposures. In particular, the drug uptake may vary up to 5- to 10-fold between fasted and fed state, depending on the fat content of the meal.

Furthermore, the low solubility of Zytiga^{®} in water is one of the factors leading to a low bioavailability of abiraterone acetate in the organism. The absolute bioavailability of Zytiga^{®} is reported to be no more than 10%, as the drug is mainly metabolized to abiraterone (free base) and then excreted by feces (-88%) and urine (-5%) with a terminal half-life of 15 hours. This means that from a daily dose of Zytiga^{®} (1000 mg in the form of 250 mg tablets x4 times per day, or 500 mg tablets x 2 times per day), only 10% of the drug can develop a therapeutic effect (100 mg). In addition to this low bioavailability, abiraterone acetate presents an intra and inter variability of absorption between subjects. Further, due to the exceptionally high dose, Zytiga^{®} tablet size is quite large.

Yonsa^{®}, is an abiraterone acetate nanosized formulation comprising abiraterone acetate that is given as an oral daily 500 mg dose (four 125 mg tablets) instead of an oral daily dose of 1000 mg. This nanosized formulation is obtained by a micronization dry-milling technology developed by Iceutica (WO2014145813 and WO2016044701). This technology is, however, linked to a specific particle size of the active ingredient in the range of the nanometer scale (100 to 500 nanometers). Nevertheless, these nanoparticles can easily agglomerate which can cause problems in the final formulation (such as stability) and manufacturing. Thus, new formulations wherein abiraterone does not need to be nanosized are desirable. Further, the treatment with Yonsa^{®} has the disadvantage that still 4 tablets of abiraterone acetate 125 mg daily should be administered. Taking into consideration that abiraterone acetate treatment is generally combined with prednisone, prednisolone or methylprednisolone, patients under this therapy are faced with taking several tablets per day during a long period of time. In addition, metastatic castration-resistant prostate cancer is generally diagnosed to elder people, who generally present swallowing problems and can easily forget their medication schedule. Moreover, complex medication regimens lead to patient non medication adherence. Adherence to therapies is a primary determinant of treatment success. Failure to adherence is a serious problem which not only affects the patient but also the healthcare system. Medication nonadherence in patients leads to substantial worsening of disease, death and increased health care costs.

So, there is a benefit on developing a low total daily amount of abiraterone, abiraterone acetate or pharmaceutically acceptable salts thereof that may allow an effective treatment of prostate cancer patients, decrease the intra-inter variability between subjects and reduce the effect of food. Also, there is a need to reduce the number of tablets of abiraterone acetate to be administered to patients, while maintaining an effective treatment. Specifically, for older people there is a need to reduce the size and the number of tablets to be administered.

### SUMMARY OF THE INVENTION

The first aspect of the present invention is abiraterone, abiraterone acetate, or pharmaceutically acceptable salts thereof, as active ingredient, for use in a method for treating cancer, wherein the method comprises the administration, for at least more than one day, of a total daily amount of the active ingredient from 25 mg to 210 mg equivalent to abiraterone acetate, preferably throughout the administration of an immediately release pharmaceutical composition.

A second aspect of the present invention provides a pharmaceutical composition comprising the abiraterone, abiraterone acetate, or pharmaceutically acceptable salts for use in a method for treating cancer, wherein the method comprises the administration for at least more than one day of a total daily amount of the active ingredient from 25 mg to 210 mg equivalent to abiraterone acetate, preferably throughout the administration of an immediately release pharmaceutical composition.

A third aspect of the present invention deals with a process for the preparation of the said pharmaceutical composition.

### DESCRIPTION OF THE INVENTION

It is the object of the present invention to reduce the necessary daily dose of abiraterone acetate. It is an additional object of the invention to reduce the food effect of abiraterone acetate and decrease patients intra-inter variability.

### Definitions

As used in the present disclosure, the following words, phrases, and symbols are generally intended to have the meanings as set forth below, except to the extent that the context in which they are used indicates otherwise.

The term "active ingredient" refers to a therapeutically active compound, as well as any prodrugs thereof and pharmaceutically acceptable salts, hydrates and solvates of the compound and the prodrugs. Disclosed compositions of the present invention may comprise as active ingredient abiraterone, abiraterone acetate, or a pharmaceutically acceptable salts and can include further active ingredients in the same or in a separate composition.

Abiraterone, abiraterone acetate or pharmaceutically acceptable salts thereof can be administered in combination with further active ingredients such as poly(ADP-ribose) polymerase (PARP) inhibitors (such as olaparib, veliparib, iniparib, niraparib, rucaparib); trastuzumab; lapatinib; carboplatin; taxane; gemcitabine; epirubicin; apatinib; cediranib; capecitabine; 7-hydroxystaurosporine (UCN- 01); bortezomib; denaciclib; panobinostat; dasatinib; enzalutamide, apalutamide, bicalutamide, flutamide, nilutamide, ketonazole, orteronel, finasteride, dutasteride, bexlosteride, izonsteride, turosteride, episteride, dexamethasone, leuprolide, goserelin, triptorelin, histrelin, estrogen, olasertib, rigosertib prednisone, prednisolone, methylprednisolone, pharmaceutically acceptable salts, acids, or derivatives of any of the above and mixtures thereof.

Preferably, abiraterone, abiraterone acetate or salts thereof can be administered in combination with dutasteride, enzalutamide, apalutamide, bicalutamide, docetaxel or niraparib or mixtures thereof.

The term "abiraterone pharmaceutically acceptable salts" refers to any salt such as acetates, sulfates, pyrosulfates, bisulfates, sulfites, bisulfites, phosphates, monohydrogen phosphates, dihydrogenphosphates, metaphosphates, pyrophosphates, chlorides, bromides, iodides, propionates, decanoates, caprylates, acrylates, formates, isobutyrates, caproates, heptanoates, propiolates, oxalates, malonates, succinates, suberates, sebacates, fumarates, maleates, butyne-1 ,4-dioates, hexyne-1 ,6-dioates, benzoates, chlorobenzoates, methylbenzoates, dinitrobenzoates, hydroxybenzoates, methoxybenzoates, phthalates, sulfonates, xylenesulfonates, phylacetates, phenylpropionates, phenylbutyrates, citrates, lactates, gammahydroxy-butyrates, glycolates, tartrates, alkanesulfonates (e.g. methanesulfonate or mesylate), propanesulfonates, naphthalene-1 -sulfonates, naphthalene-2-sulfonates, or mandelates.

Abiraterone, abiraterone acetate or pharmaceutically acceptable salts thereof as active ingredient or in the pharmaceutical formulation can be in a crystalline or non-crystalline solid form.

As used herein, the term "cancer" refers to the growth, division or proliferation of abnormal cells in the body. Preferably, the total daily amount of abiraterone, abiraterone acetate, or pharmaceutically acceptable salts thereof of the present invention is used in cancer patients that does not develop hepatotoxicity during the treatment.

Cancers that can be treated with the methods and the compositions described herein include, but are not limited to, prostate cancer, metastatic prostate cancer, castration-resistant prostate cancer, metastatic castration-resistant prostate cancer, hormone-sensitive prostate cancer, metastatic hormone-sensitive prostate cancer, breast cancer, triple negative breast cancer, adrenal cancer, leukemia, lymphoma, myeloma, Waldenstrom's macroglobulinemia, monoclonal gammopathy, benign monoclonal gammopathy, heavy chain disease, bone and connective tissue sarcoma, brain tumors, thyroid cancer, pancreatic cancer, pituitary cancer, eye cancer, vaginal cancer, vulvar cancer, cervical cancer, uterine cancer, ovarian cancer, polycystic ovarian cancer, esophageal cancer, stomach cancer, colon cancer, rectal cancer, liver cancer, gallbladder cancer, cholangiocarcinoma, lung cancer, testicular cancer, penal cancer, oral cancer, skin cancer, kidney cancers, Wilms' tumor, bladder cancer or any combination thereof.

Preferably, the treated cancer with the total daily amount of abiraterone, abiraterone acetate, or pharmaceutically acceptable salts thereof of the present invention is prostate cancer. More preferably metastatic prostate cancer, castration-resistant prostate cancer, hormone-sensitive prostate cancer or any combination thereof. Even more preferably, metastatic castration-resistant prostate cancer, metastatic hormone-sensitive prostate cancer or any combination thereof.

As used herein, the term "total daily amount" refers to the maximum total daily dose equivalent to abiraterone acetate that is administered to a patient suffering from cancer, preferably prostate cancer, more preferably metastatic prostate cancer.

The total daily amount is administered for at least more than one day. For the sake of clarity "at least more than one day" refers to the total daily dosage amount that is administered continuously over a period of time sufficient to treat the cancer. Preferably at least 7 days. More preferably, at least for 1 month. Even more preferably, at least for 6 months.

As used herein, the term "immediate release pharmaceutical composition" refers to a pharmaceutical composition having a dissolution performance such as 60 % or more of the active ingredient contained in said pharmaceutical composition dissolves within 20 minutes (min).

As used herein, the term "unit dosage form" refers to a physically discrete unit that contains a predetermined quantity of the active ingredient calculated to produce a desired therapeutic effect daily. Unit dosage forms refers to the number of units needed for a daily administration.

A first aspect of the invention relates to abiraterone, abiraterone acetate, or pharmaceutically acceptable salts thereof, as active ingredient, for use in a method for treating cancer, wherein the method comprises the administration for at least more than one day of a total daily amount of the active ingredient from 25 mg to 210 mg equivalent to abiraterone acetate, preferably throughout the administration of an immediately release pharmaceutical composition.

In a preferred embodiment, the total daily amount of abiraterone, abiraterone acetate or pharmaceutically acceptable salts thereof, for use in a method for treating cancer is from 50 mg to 190 mg. More preferably from 100 mg to 160 mg. Even more preferably from 80 mg to 130 mg.

In another embodiment, the total daily amount of abiraterone, abiraterone acetate or pharmaceutically acceptable salts thereof for use in a method for treating cancer is preferably from 25 mg to 120 mg, 50 mg to 120 mg, 60 mg to 120 mg, 50 to 125 mg or 60 mg to 120 mg. Preferably, from 25 mg to 30 mg, 30 mg to 40 mg, 40 mg to 50 mg, 50 mg to 60 mg, 60 mg to 70 mg, 70 mg to 80 mg, 80 mg to 90 mg, 90 mg to 100 mg, 100 mg to 110 mg, 110 mg to 120 mg, 120 mg to 130 mg, 130 mg to 140 mg, 140 mg to 150 mg, 150 mg to 160 mg, 160 mg to 170 mg, 170 mg to 180 mg, 180 mg to 190 mg, 190 mg to 200 mg or 200 mg to 210 mg.

Preferably, the total daily amount is administered in the form of an immediate release pharmaceutical composition. More preferably, the said immediate release pharmaceutical composition releases at least 60 % of the active ingredient in 20 min.

According to another embodiment, the said total daily amount of the active ingredient is used for a method for treating cancer, preferably prostate cancer, more preferably metastatic prostate cancer.

In an embodiment, the said total daily amount of the active ingredient for use in a method for treating cancer can be administered with or without food.

In a further embodiment, the total daily amount of abiraterone, abiraterone acetate, or a pharmaceutically acceptable salts thereof for use in a method for treating cancer can be administered in one or more unit/s dosage forms. Preferably, the said total daily amount is administered in less than three units dosage forms. More preferably, it is administered in two units dosage forms. Even more preferably, it is administered in one unit dosage form.

In a further embodiment, the said one or more unit/s dosage forms comprise/s between 10% to 80% (w/w) weight of the active ingredient expressed as abiraterone acetate, in regard to the fill mixture weight of the unit dosage form. Preferably, between 10% to 45%. More preferably, between 10% to 30% (w/w). More preferably, between 15% to 30% (w/w). Even more preferably, between 15% to 25% (w/w).

The obtained unit/s dosage forms are smaller than Zytiga^{®} tablets. Further, the number of the units dosage forms to be administered are reduced in comparison with Zytiga^{®} tablets.

In a second aspect, the invention relates to a pharmaceutical composition comprising the said total daily amount of abiraterone, abiraterone acetate, or pharmaceutically acceptable salts thereof, for use in a method for treating cancer, during at least more than one day.

In an embodiment, the said pharmaceutical composition can be in the form of immediate or extended release. Preferably, the said pharmaceutical composition is in the form of immediate release.

The pharmaceutical composition comprising said total daily amount of abiraterone, abiraterone acetate or pharmaceutically acceptable salts thereof is bioequivalent to Zytiga^{®} and further exhibits an increased bioavailability in comparison to Zytiga^{®}.

By bioequivalent is it meant that the mean area under the curve (AUC₀₋ₜ) and/or the mean maximum serum concentration (Cₘₐₓ) of the pharmaceutical composition comprising the total daily amount of abiraterone, abiraterone acetate or pharmaceutically acceptable salts thereof of the present invention is in the range of 80% to 125% of the mean AUC₀₋ₜ or mean Cₘₐₓ of reference Zytiga^{®}.

In some embodiments, the ratio between the arithmetic Cₘₐₓ and AUC₀₋ₜ of the pharmaceutical composition comprising the total daily amount of abiraterone, abiraterone acetate or pharmaceutically acceptable salts thereof of the present invention can be comprised between 1:10 and 3:10. Preferably, between 3:2 and 5:2. More preferably, between 8:5 and 4:2.

In some embodiments, the AUC of the pharmaceutical composition of the present invention divided by the total daily amount administered to the patient can be at least 3-fold the AUC of the reference Zytiga^{®}, divided by the total daily amount administered of the reference Zytiga^{®} (1000 mg). Preferably, it can be at least 5-fold. More preferably, it can be at least 6-fold. More preferably it can be at least 7-fold. Even more preferably it can be at least 8-fold. Even more preferably, it can be at least 10-fold.

The pharmaceutical composition of the present invention can be administered in any conventional form and route known in the art including, but not limited to intravenous, oral, rectal, aerosol, parenteral, ophthalmic, pulmonary, transdermal, vaginal, otic, nasal, and topical administration.

The pharmaceutical composition of the present invention comprising the total daily amount of the active ingredient may further comprise one or more pharmaceutically acceptable excipients.

The excipients may be selected from conventional pharmaceutical excipients such as surfactants, fillers, diluents, lubricants, binders, disintegrating agents, colorants, flavoring agents, sweeteners, glidants, preservatives, stabilizers, antioxidants, or buffers.

In a preferred embodiment of the second aspect of the invention, the pharmaceutical composition comprises one or more surfactant/s. Preferably, the pharmaceutical composition comprises at least one surfactant or at least two surfactants or at least three surfactants. More preferably, the surfactant or surfactants are selected from non-ionic, ionic and mixtures thereof.

Typical ionic surfactants are, for example, octenidine dihydrochloride, cetrimonium bromide, cetylpyridinium chloride, benzalkonium chloride, benzethonium chloride, dimethyl dioctadecyl ammonium chloride, dioctadecyl dimethyl ammonium bromide, sodium dodecyl sulfate or sodium lauryl ether sulfate or mixtures thereof.

Non-ionic surfactants are considered, for example, as polyoxyethylene products of hydrogenated vegetable oils, polyoxyethylene-sorbitan-fatty acid esters, polyoxyethylene castor oil derivatives, sorbitan esters (e.g. Tween, Span), castor oil derivatives (e.g. Cremophor EL, Cremophor RH40, Cremophor RH60), macrogol 15 hydroxystearate (Solutol HS-15, Kolliphor HS-15), polyvinyl caprolactam-polyvinyl acetate-polyethylene glycol graft copolymer (Soluplus), glyceryl monolinoleate (Maisine), sucrose esters (e.g. sucrose palmitate), poloxamers, polyglycolyzed glycerides (e.g. caprylocaproyl macrogol-8 glyceride (Labrasol or Labrasol ALF), Stearoyl macrogol-32 glyceride (Labrafac), linoleoyl macrogol-6 glycerides (Labrafil, such as Labrafil 1944 CS, Labrafil 2125 CS or Labrafil 2130 CS), polyglyceryl-3 dioleate (Plurol oleique such as Plurol oleique CC 497), polyglyceryl-3 diisostearate (Plurol diisostearique)), lauroyl polyoxyl/macrogol 32 glycerides (Gelucire), propylene glycol monocaprylate (Cs) esters (Capryol 90), propylene glycol monolaurate (Lauroglycol FCC or Lauroglycol 90), glyceryl monooleate (Peceol) mono, di, tricaprylic (Cs), capric acid (Cia), Polyoxylethylene stearates (Tefose 63 or Tefose 1500), Soybean oil Glyceryl distearate polyglyceryl-3 oleate (Geloil SC) Polyoxyl 40 hydrogenated castor oil (Kolliphor RH40), lecithin, diethylene glycol monoethyl ether (Transcutol) or mixtures thereof.

In a preferred embodiment, the pharmaceutical composition comprises one or more surfactant/s. Preferably, the one or more surfactant/s are non-ionic.

In a preferred embodiment, the non-ionic surfactant/s are selected from polyglycolyzed glycerides (caprylocaproyl macrogol-8 glyceride (Labrasol or Labrasol ALF), Stearoyl macrogol-32 glyceride (Labrafac), linoleoyl macrogol-6 glycerides (Labrafil, such as Labrafil 1944 CS, Labrafil 2125 CS or Labrafil 2130 CS)), polyglyceryl-3 dioleate (Plurol oleique such as Plurol oleique CC 497), propylene glycol monocaprylate (Cs) esters (Capryol 90), Propylene glycol monolaurate (Lauroglycol) or mixtures thereof.

More preferably, the non-ionic surfactant/s are selected from the polyglycolyzed glycerides (caprylocaproyl macrogol-8 glyceride (Labrasol or Labrasol ALF), Stearoyl macrogol-32 glyceride (Labrafac), linoleoyl macrogol-6 glycerides (Labrafil such as Labrafil 1944 CS, Labrafil 2125 CS or Labrafil 2130 CS)), polyglyceryl-3 dioleate (Plurol oleique such as Plurol oleique CC 497) or mixtures thereof.

Even more preferably, the non-ionic surfactant/s are selected from caprylocaproly macrogol glyceride (Labrasol or Labrasol ALF), linoleaoyl macrogol glycerides (Labrafil such as Labrafil 1944 CS, Labrafil 2125 CS or Labrafil 2130 CS), polyglyceryl-3 dioleate (Plurol oleique such as Plurol oleique CC 497) or mixtures thereof.

The total daily amount of the surfactants in the pharmaceutical composition of the invention can be between 20% to 90% by weight (w/w) in regard to the fill mixture weight composition. Preferably, between 55% to 90% (w/w). More preferably, between 70% to 90% (w/w). More preferably, between 70% to 85% (w/w). Even more preferably, between 75% to 85% (w/w).

In another embodiment, the pharmaceutical composition of the invention further comprises an antioxidant. The antioxidant can be selected from the group of free radical absorbers (such as: vitamin E, class Carotene), oxygen scavengers (such as: carotenoids and their derivatives, ascorbic acid, ascorbyl palmitate, isoascorbic acid Acid, sodium erythorbate), metal ion chelating agents (such as citric acid, EDTA and phosphoric acid derivatives), butylhydroxytoluene (BHT), butyl hydroxyanisole (BHA), sodium sulfite, sodium metabisulfite, propyl gallate, alpha tocopherol or any mixtures thereof. Preferably, the antioxidant is BHT.

Suitable amounts of antioxidants range from 0.0010% to 1% (w/w), preferably from 0.0025 to 0.5% (w/w), more preferably from 0.005 to 0.2% (w/w).

Additionally, the pharmaceutical composition of the invention may further comprise other pharmaceutical excipients such as fillers, diluents, lubricants, binders, disintegrating agents, colorants, flavoring agents, sweeteners, glidants, preservatives, stabilizers or buffers.

In a preferred embodiment, the pharmaceutical composition is administered in an oral form. Said oral form may be selected from the group selected from capsules (e.g. soft gelatin capsules, liquid-filled hard capsules (hard gelatin capsules or hydroxypropylmethyl cellulose capsules)), cachets, pills, tablets, lozenges (using a flavored basis, usually sucrose and acacia or tragacanth), powders, granules, cores, pellets, beads, sachets, or as a solution or a suspension in an aqueous or nonaqueous liquid, or as an oil-in-water or water-in-oil liquid emulsion, or as an elixir, or sticks or syrup or as mouthwashes and the like.

In a preferred embodiment, the pharmaceutical composition of the present invention is a tablet or a capsule. More preferably, it is a capsule. Even more preferably, it is a liquid-filled hard capsule or a soft gelatin capsule.

Capsules show better patient compliance, greater flexibility in dosage form design, and less expensive manufacturing process than other oral solid forms.

Capsules can be filled with liquids (solutions, suspensions, emulsions...) or semisolids. Liquid-filled capsules can be hard or soft in form and may conveniently deliver the active ingredient within a liquid formulation. The fill formulation can contain either solubilized or suspended active ingredients. Even if the active ingredient is suspended, an efficient total daily amount formulation of abiraterone, abiraterone acetate or abiraterone pharmaceutically acceptable salts can be obtained.

Hard capsules may be filled with powder, granules or beads. Alternatively, hard capsules may be filled with liquids or semisolids. Soft gelatin capsules may be filled with liquids or semisolids.

Optionally, the composition of the present invention may be adsorbed onto the surface of carriers and filed into capsules or compressed, using conventional techniques (for instance, wet and dry granulation, hot melt granulation) to form tablets.

As carriers, polymers can be used, in particular water soluble polymers such as hydroxypropyl methyl cellulose (HPMC), hydroxypropyl methyl cellulose (HPMC), hydroxypropylmethyl cellulose acetate succinate (HPMCAS), hydroxypropyl methyl cellulose acetate trimellitate (HPMCAT), carboxymethylcellulose acetate butyrate (CMCAB), hydroxypropylmethyl cellulose acetate succinate (HPMCAS), polyvinylpyrrolidone, polyethyleneglycol (PEG) or poly(meth)acrylates (Eudragit).

Further, inorganic carriers can also be used, such as silica or silica derivatives as mesoporous silica (such as Syloid FP or Syloid XDP), hydrophilic fumed silica (such as Aerosil) or silicates (such as aluminium silicate or aluminometasilicate (MAS)).

In a third aspect, the invention relates to a process for the manufacture of the pharmaceutical composition of the invention.

Pharmaceutical compositions of the present invention may be formulated in conventional manner using one or more physiologically acceptable carriers comprising excipients and auxiliaries which facilitate processing the active compounds into preparations which can be used pharmaceutically. Such conventional manufacturing techniques are, for instance, mixing, dissolving, granulating, dragee-making, levigating, emulsifying, encapsulating, entrapping or compression processes.

The formulations of the invention are stable and easy to manufacture in a simple, robust and reproducible way.

In an embodiment of the third aspect, the process for the manufacture of the pharmaceutical composition of the invention comprises the following steps:
(i) stirring of at least one surfactant and, optionally, other excipients in a suitable container;
(ii) adding to (i) the total daily amount of abiraterone, abiraterone acetate or pharmaceutically acceptable salt thereof;
(iii) stirring the resulting fill mixture until complete homogenization.

In another embodiment, the process for the manufacture of the pharmaceutical composition of the invention comprises the following steps:
(i) stirring of at least one surfactant and, optionally, other excipients in a suitable container;
(ii) adding to (i) the total daily amount of abiraterone, abiraterone acetate or pharmaceutically acceptable salt thereof;
(iii) stirring the resulting fill mixture until complete homogenization;
(iv) encapsulating the mixture of step (iii).

In another embodiment, the process for the manufacture of the pharmaceutical composition of the invention may further comprise a step of sealing the capsule with a gelatin mass. Thus, the said process may comprise the following steps:
(i) stirring of at least one surfactant and, optionally, other excipients in a suitable container;
(ii) adding to (i) the total daily amount of abiraterone, abiraterone acetate or pharmaceutically acceptable salt thereof;
(iii) stirring the resulting fill mixture until complete homogenization;
(iv) encapsulating the mixture of step (iii);
(v) optionally, sealing the capsule of step (iv) with a banding mass.

In a further embodiment the banding mass comprises suitable excipients selected from gelatin, HPMC or HPC. Optionally, the said banding mass may further contain other excipients such as colorants or other.

The said banding mass may be prepared following conventional procedures in the art. As an example, gelatin, HPMC or HPC may be stirred in water, optionally with a colorant, at a desired temperature, until complete homogenization.

In another embodiment of the third aspect of the invention, the process for the manufacture of the pharmaceutical composition of the invention comprises the following steps:
(i) stirring of at least one surfactant and, optionally, other excipients in a suitable container;
(ii) adding to (i) the total daily amount of abiraterone, abiraterone acetate or pharmaceutically acceptable salt thereof;
(iii) stirring the resulting fill mixture until complete homogenization;
(iv) preparing a shell mass;
(v) encapsulating the mixture of step (iii) with the shell mass of step (iv).

In a further embodiment, the shell mass comprises gelatin and, optionally other excipients such as plasticizers like glycerine, glycerol, soy lecithin or triglycerides, water, opacifiers, colorants like TiO₂ or carmoisine, flavours, sweeteners and/or preservatives like potassium sorbate, methyl, ethyl, or propyl hydroxybenzoate.

The said shell mass may be prepared following conventional procedures in the art. As an example, gelatin and other excipients may be stirred in water at a desired temperature, until complete homogenization.

In a further embodiment of the third aspect, the process for the manufacture of the pharmaceutical composition of the invention comprises the following steps:
(i) stirring of at least one surfactant and, optionally, other excipients in a suitable container;
(ii) adding to (i) the total daily amount of abiraterone, abiraterone acetate or pharmaceutically acceptable salt thereof;
(iii) stirring the resulting fill mixture until complete homogenization;
(iv) adding a carrier and, optionally, adding further excipients;
(v) optionally, encapsulating the resulting mixture of step (iv)

In a further embodiment of the third aspect, the process for the manufacture of the pharmaceutical composition of the invention comprises the following steps:
(i) stirring of at least one surfactant and, optionally, other excipients in a suitable container;
(ii) adding to (i) the total daily amount of abiraterone, abiraterone acetate or pharmaceutically acceptable salt thereof;
(iii) stirring the resulting fill mixture until complete homogenization;
(iv) adding a carrier and, optionally, adding further excipients;
(v) optionally, compressing the resulting mixture of step (iv).

### Preferred embodiments of the invention

Each of the aspects and embodiments of the present invention described herein may be combined with one or more aspects and embodiments of the present invention.

A preferred embodiment of the present invention relates to a pharmaceutical oral form composition comprising the active ingredient abiraterone, abiraterone acetate, or pharmaceutically acceptable salts for use in a method for treating cancer, wherein the method comprises:
i) the administration for at least more than one day of a total daily amount of the active ingredient from 50 mg to 190 mg equivalent to abiraterone acetate, throughout the administration of an immediately release pharmaceutical composition such that more than 60% is released within 20 minutes;
ii) wherein the oral form is administered with or without food;
iii) wherein the oral form is administered in less than two unit dosage forms comprising between 10% to 45% weight of the active ingredient;
iv) and wherein the oral form is a tablet or a capsule.

A preferred embodiment of the present invention relates to a pharmaceutical oral form composition comprising the active ingredient abiraterone, abiraterone acetate, or pharmaceutically acceptable salts for use in a method for treating cancer, wherein the method comprises:
i) the administration for at least more than one day of a total daily amount of the active ingredient from 50 mg to 190 mg equivalent to abiraterone acetate, throughout the administration of an immediately release pharmaceutical composition such that more than 60% is released within 20 minutes;
ii) wherein the oral form is administered in less than two unit dosage forms comprising between 10% to 45% weight of the active ingredient;
iii) wherein the oral form comprises one or more surfactants, preferably selected from linoleoyl macrogol-6 glycerides (Labrafil such as Labrafil 1944 CS, Labrafil 2125 CS or Labrafil 2130 CS), caprylocaproyl macrogol-8 glyceride (Labrasol or Labrasol ALF), polyglyceryl3 dioleate (Plurol oleique such as Plurol oleique CC 497) or mixtures thereof;
iv) and wherein the oral form optionally comprises an antioxidant, preferably BHT.

Another preferred embodiment of the present invention relates to a pharmaceutical composition comprising the active ingredient abiraterone, abiraterone acetate, or pharmaceutically acceptable salts thereof, for use in a method for treating cancer, wherein the method comprises:
i) the administration for at least more than one day of a total daily amount of the active ingredient from 50 mg to 190 mg equivalent to abiraterone acetate, throughout the administration of an immediately release pharmaceutical composition such that more than 60% is released within 20 minutes;
ii) wherein the pharmaceutical composition is administered in less than two unit dosage forms comprising between 10% to 45% weight of the active ingredient;
iii) wherein the pharmaceutical composition is bioequivalent to Zytiga^{®};
iv) wherein the ratio between the arithmetic Cₘₐₓ and AUC₀₋ₜ of the pharmaceutical composition is comprised between 8:5 and 4:2;
v) and wherein the AUC of the pharmaceutical composition of the present invention divided by the total daily amount administered to the patient is at least 6-fold the AUC of the reference Zytiga^{®} divided by the total daily amount administered of the reference Zytiga^{®} (1000 mg).

Although the present invention has been described in terms of a limited number of embodiments, benefiting from the above description, the skilled in the art understand that other embodiments are conceivable within the scope of the invention thus described.

### EXAMPLES

The examples, which are incorporated and form part of the specification, merely illustrate certain embodiments of the present invention and should not be construed as limiting the invention. They are meant to serve to explain specific modes of the present invention to those skilled in the art

### GENERAL PROCESS FOR LIQUID FILLED HARD CAPSULES EXAMPLES 1 TO 10:

Abiraterone acetate used in the following examples presents a D50 particle between 3 and 10 microns and D90 between 3 and 20.

### 1. Preparation of abiraterone acetate fill mixture:

For each example, the corresponding quantity of surfactants of table 1 is added into a container of suitable capacity under continuous agitation. In example 9, an antioxidant is further added. Abiraterone acetate is added to the container and stirred until complete homogenization, for at least 30 minutes, at room temperature (T=20-25°C) .

### 2. Capsules filling:

Empty capsules are filled with the abiraterone acetate fill mixture prepared in the step 1 using a pipette.

### 3. Preparation of banding mass:

500 mL of purified water is added into a stainless-steel container of suitable capacity. 155 g of gelatin are added and stirred with a metal spatula to ensure that the gelatin is wetted. The vessel is covered and left to stand for one hour or until complete hydration. The container is placed in an oven or water bath set to 55 ± 10°C until complete dissolution of the gelatin.

Final viscosity of the gelatin mass is between 186 and 225 CPs.

### 4. Capsules banding:

The banding mass of step 3 is applied to the capsules filled in the step 2 using a semi automatic capsule band-sealer machine.

### EXAMPLES TABLE 1 TO 10

| Excipients | Examples (mg/capsule) | | | | | | | | | |
|---|---|---|---|---|---|---|---|---|---|---|
| | 1 Z1910 | 2 Z1939 | 3 Z1941 | 4 Z1945 | 5 Z1948 | 6 Z1949 | 7 Z2006 | 8 Z2007 | 9 Z2008 | 10 Z2009 |
| Abiraterone Acetate | 62.5 | 62.5 | 62.5 | 115.1 | 62.5 | 125 | 62.5 | 62.5 | 62.5 | 62.5 |
| Labrasol | 362.6 | 129.2 | 119.3 | 256.3 | 174.0 | 348.0 | 348.0 | - | - | - |
| Labrasol ALF | - | - | - | - | - | - | - | 172.3 | 172.3 | 87.0 |
| Labrafil | - | 43.5 | - | 48.5 | 32.9 | 65.8 | 65.8 | 32.9 | 32.9 | 16.5 |
| Capryol 90 | - | 17.6 | 52.9 | - | - | - | - | - | - | - |
| Lauroglycol | - | - | 17.0 | - | - | - | - | - | - | - |
| Plurol oleique | - | - | - | 50.5 | 34.3 | 68.5 | 68.5 | 34.3 | 34.3 | 17.1 |
| BHT | - | - | - | - | - | - | - | - | 0.03 | - |

Compositions of examples 1 to 10 are stable, have a suitable uniformity of dose unit, present suitable dissolution profiles and desirable pharmacokinetic properties.

## Claims

1. Abiraterone, abiraterone acetate, or pharmaceutically acceptable salts thereof, as active ingredient, for use in a method for treating cancer, wherein the method comprises the administration for at least more than one day of a total daily amount of the active ingredient from 25 mg to 210 mg equivalent to abiraterone acetate, preferably throughout the administration of an immediately release pharmaceutical composition.

2. The abiraterone, abiraterone acetate, or pharmaceutically acceptable salts thereof, for use according to claim 1, wherein the administration of a total daily amount of the active ingredient is from 50 mg to 190 mg equivalent to abiraterone acetate.

3. The abiraterone, abiraterone acetate, or pharmaceutically acceptable salts thereof, for use according to any of claims 1 to 2, wherein the administration of a total daily amount of the active ingredient is from 100 mg to 160 mg equivalent to abiraterone acetate.

4. The abiraterone, abiraterone acetate, or pharmaceutically acceptable salts thereof, for use according to any of the preceding claims, wherein the cancer is a prostate cancer, preferably metastatic prostate cancer.

5. The abiraterone, abiraterone acetate, or pharmaceutical acceptable salts thereof, for use according to any of the preceding claims wherein the total daily amount is administered with or without food.

6. The abiraterone, abiraterone acetate, or pharmaceutically acceptable salts thereof, for use according to any of the preceding claims wherein the total daily amount is administered in one or more unit/s dosage forms.

7. The abiraterone, abiraterone acetate, or pharmaceutical acceptable salts thereof, for use according to any of the preceding claims wherein the unit/s dosage forms comprise/s between 10% to 80% weight of the active ingredient.

8. The abiraterone, abiraterone acetate, or pharmaceutically acceptable salts thereof, for use according to claim 7 wherein the unit/s dosage forms comprise/s between 10% to 45% weight of the active ingredient.

9. The abiraterone, abiraterone acetate, or pharmaceutically acceptable salts thereof, for use according to any of the claims 7 to 8 wherein the unit/s dosage forms comprise/s between 10% to 30% weight of the active ingredient.

10. A pharmaceutical composition comprising the abiraterone, abiraterone acetate, or pharmaceutically acceptable salts for use according to any of the preceding claims.

11. The pharmaceutical composition according to claim 10, which is an immediate release composition.

12. The pharmaceutical composition according to any of the claims 10 to 11, comprising one or more surfactant/s, preferably wherein the one or more surfactant/s are non-ionic, more preferably wherein the non-ionic surfactant/s are selected from polyglycolyzed glyceride (caprylocaproyl macrogol-8 glyceride (Labrasol or Labrasol ALF), Stearoyl macrogol-32 glyceride (Labrafac), linoleoyl macrogol-6 glycerides (Labrafil such as Labrafil 1944 CS, Labrafil 2125 CS or Labrafil 2130 CS)), polyglyceryl-3 dioleate (Plurol oleique such as Plurol oleique CC 497), propylene glycol monocaprylate (Cs) ester (Capryol 90), propylene glycol monolaurate (Lauroglycol) or mixtures thereof; even more preferably from polyglycolyzed glyceride (caprylocaproyl macrogol-8 glyceride (Labrasol or Labrasol ALF), linoleoyl macrogol-6 glycerides (Labrafil such as Labrafil 1944 CS, Labrafil 2125 CS or Labrafil 2130 CS)), polyglyceryl3 dioleate (Plurol oleique such as Plurol oleique CC 497) or mixtures thereof.

13. The pharmaceutical composition according to claim 12, further comprising an antioxidant, preferably selected from the group of free radical absorbers (such as: vitamin E, class Carotene), oxygen scavengers (such as: carotenoids and their derivatives, ascorbic acid, ascorbyl palmitate, isoascorbic acid Acid, sodium erythorbate), metal ion chelating agents (such as citric acid, EDTA and phosphoric acid derivatives), butylhydroxytoluene (BHT), butyl hydroxyanisole (BHA), sodium sulfite, sodium metabisulfite, propyl gallate, alpha tocopherol or mixtures thereof; more preferably, wherein the antioxidant is BHT.

14. The pharmaceutical composition according to any of the claims 10 to 13 which is in an oral form, preferably, the oral form is a tablet or a capsule.

15. A process for the preparation of the pharmaceutical composition according to any of the claims 10 to 14 comprising the steps of:
(i) stirring of at least one surfactant and, optionally, other excipients in a suitable container;
(ii) adding to (i) the total daily amount of abiraterone, abiraterone acetate or pharmaceutically acceptable salt thereof;
(iii) stirring the resulting fill mixture until complete homogenization.
